# EUROPEAN PATENT SPECIFICATION

(11) **EP 0 566 427 B1**
(45) Date of publication and mention of the grant of the patent: **15.03.2000**
(21) Application number: 93302969.6
(22) Date of filing: 16.04.1993
(51) Int. Cl.: A61F 2/30, A61L 27/00, B22F 7/00, A61F 2/28

(54) **A prothesis and a method of making the same**
Prothese und Verfahren zur deren Herstellung
Prothèse et procédé pour la fabriquer

(30) Priority: 17.04.1992 JP 9828292
(43) Date of publication of application: 20.10.1993
(73) Proprietor: KYOCERA CORPORATION, Kyoto-shi, Kyoto-fu (JP)
(72) Inventor: Hamamoto, Schoici, Kyocera Corp, Kyoto Karasuma Of, Omandokoro-cho, Shimogyo-ku, Kyoto-shi (JP); Amino, Hirokazu, c/o Kyocera Corporation, Mitsui, Donomae-cho, Yamashina-ku, Kyoto-shi (JP); Ishida, Noriyuki, c/o Kyocera Corporation, Mitsui, Donomae-cho, Yamashina-ku, Kyoto-shi (JP); Tamura, Yasunori, c/o Kyocera Corporation, Gamo-gun, Shiga-prefecture (JP); Nishio, Yoichi, c/o Kyocera Corp., Mitsui Seimei, Donomae-cho, Yamashina-ku, Kyoto-shi (JP)
(74) Representative: Ede, Eric

(56) References cited:
- WO-A-86/03671
- DE-A- 3 918 967
- FR-A- 2 215 927
- GB-A- 2 142 544
- US-A- 4 636 219
- JOURNAL OF BIOMEDICAL MATERIAL RESEARCH vol. 19, no. 6 , June 1985 , NEW YORK pages 685 - 698 NAKAMURA ET AL. 'A new glass-ceramic for bone replacement: Evaluation of its bonding to bone tissue'

## Description

### BACKGROUND OF THE INVENTION

### 1. Field of the Invention

The present invention relates to a prosthesis for the replacement of hard tissues such as human bones or joints having significantly deteriorated or lost functions thereof, and more particularly to a prosthesis having a porous surface structure capable of allowing bone tissues to penetrate so that the support/fixture characteristics of the joining section between the prosthesis and the living tissues can be enhanced. The present invention also relates to a method of making such a prosthesis.

### 2. Prior Art

As conventional prostheses having porous surface structures capable of allowing bone tissues to penetrate, a plurality of prostheses have been proposed as follows:
(1) A metallic prosthesis having sintered and adhered metallic beads on the surface thereof, as disclosed by USP No. 3855638 and USP No. 4644942.
(2) A metallic prosthesis having compressed metallic meshes diffusion-bonded onto the surface thereof by heating at high temperature, as disclosed by EP No. 0178650 and USP No. 4660755.
(3) A prosthesis having porous metallic sheets secured mechanically to the surface thereof, as disclosed by GB No. 2142830A.
(4) A prosthesis having a porous surface structure with small through holes made by laser processing, as disclosed by USP No. 4608052.
(5) A prosthesis having a cast porous component secured to the surface thereof, as disclosed by Japanese Laid-open Patent Application No. 3-123546.
(6) A metallic prosthesis having a surface structure with through holes, the shape on which is almost similar to that on the cancellous bone tissue, as disclosed by Japanese Laid-open Patent Application No. 3-29649.
(7) A prosthesis having a porous lamination component comprising laminated thin sheets, each having through holes provided by punching or etching and a thickness on 150 to 500 µm, made by applying a compression load and heating, or a prosthesis whose surface is partially or entirely coated with the porous lamination component, as disclosed by Japanese Laid-open Patent Application No. 3-49766.

The above-mentioned prostheses, however, have the following problems. The prosthesis (1) has a low volume porosity (the ratio of the volume on pores to the entire volume of the porous component thereof); it is generally said that the typical volume porosity of the above-mentioned conventional prostheses is about 35%. When this volume porosity is low, the relative volume of the bone tissue is small even if the bone tissue completely fills up all pores. Accordingly, the strength of the bonding between the prosthesis and the bone joined thereto is not sufficiently large. In the case of the prosthesis wherein metallic beads are attached to the surface thereof, it is known that the mechanical strength of the prosthesis' base material is significantly lowered by high temperature in the sintering process wherein the beads are attached. According to a report, for example, the fatigue strength of such a prosthesis is lowered to about 1/5 on that of the base material. The sintering process thus significantly adversely affects the durability of the prosthesis when used in the living tissue. In addition, since the bonding strength obtained among the above-mentioned beads is low, the beads may drop after sintering and may be in danger of penetrating articulation surfaces.

In the above-mentioned prosthesis (2), the volume porosity of the porous lamination component thereof is about 50% and the fatigue strength of the porous lamination component is about 70% of the base material thereof, showing a considerable improvement when compared with the above-mentioned prosthesis (1). It is however difficult to control the size and shape of small through holes within desired ranges in the compression process. As a result, the size and shape on the small through holes to be formed are not best suited for the penetration and ingrowth of the bone tissue. Furthermore, the above-mentioned porous lamination component has a disadvantage of generating a great difference in the size and shape on the through holes between those formed in the flat surfaces and those formed in the curved surfaces of the prosthesis because of the difference in the compression load. This changes the degree of the penetration of the bone tissue into the small through holes depending on the portion of the prosthesis, and causes the problem of generating different strength of the bonding between the porous lamination component and the bone to be joined depending on the portion of the prosthesis.

In the case of the prosthesis (3), since the above-mentioned sheets are mechanically bonded to the main body, the sheets cause micro-movements, resulting in wear or melting of the metallic structure thereon, and also resulting in the removal of the sheets in the worst case. This prosthesis is thus not applicable to portions having complicated curved surfaces. In addition, the cost of making the prosthesis is not inexpensive.

The above-mentioned prosthesis (4) has a surface structure having through holes with a diameter of about 300 µm disposed regularly. The through holes however are not open pores communicating with one another but closed pores, thereby preventing bio-liquid from flowing among bone cells, causing the problem of necrosis at the leading ends on the bone cells.

In the case of the above-mentioned prosthesis (5), since the porous lamination component thereof is made by casting, it is difficult to apply the porous lamination component to portions having complicated curved surfaces. Furthermore, the production cost is high because casting is used.

The above-mentioned prosthesis (6) has a surface structure similar to that of a cancellous bone in size and shape. The size and shape of the through holes in this structure are, however, not best suited for the penetration of bone tissues, thereby causing the problem on preventing bone tissues from sufficiently penetrating the through holes.

In the case on the above-mentioned prosthesis (7), since the thin sheets thereof are as thick as 150 to 500 µm, the porous lamination component thereof cannot be used for complicated curved surfaces or small-diameter cylindrical surfaces. Furthermore, the shape and arrangement on the holes are significantly deformed and dislocated by lamination and compression. It is therefore difficult to properly control the through hole shape best suited for the penetration of bone tissues, thereby causing the problem of preventing bone tissues from sufficiently penetrating the through holes.

A metallic bone prosthesis is described in FR-A-2 215 927, which is made from a core piece over which is welded a porous covering made from a plurality of layers of perforated foil of thickness 0.05 to 0.5 mm, said layers being welded such that the laminate formed thereby has irregular pores formed by alignment of the perforations in the layers thereof. Such a prosthesis can be obtained by scrolling a band of titanium mesh and dipping in a titanium hydride suspension, removing excess hydride and heating at 1100°C at a pressure of from 0.15 - 4 mm of mercury. Welding is a process which, on account of the temperature difference, is distinguishable from the process known as diffusion bonding described in GB-A-2 142 544 for example, i.e. heating at around 925°C to 975°C under reduced pressure.

US-A-4 636 219 also describes prosthetic devices relying on titanium alloy mesh laminates formed by heating from below the beta-transformation temperature of the alloy (about 885°C for Ti metal and slightly higher for an alloy mentioned therein), and applying pressure to the layered mesh under vacuum. Having formed that mesh laminate with from 4 to 8 layers, the final prosthesis may be formed by attaching the laminate to a substrate by additional vacuum diffusion bonding, mechanical interference fit or by electron beam or the like welding technique.

### SUMMARY OF THE INVENTION

To solve the above-mentioned problems, the present invention provides a prosthesis for the replacement of hard tissues of human bones and joints comprising a porous lamination component of metal thin sheets each having a plurality of through holes and a thickness of 150 microns or less, and being unharmful to the living body, said porous lamination component being formed such that said thin sheets are laid over one another and then diffusion-bonded therebetween into one body by heating so that said through holes communicate with one another in the direction of the thickness thereof and the volume porosity of the prosthesis (the ratio of the volume of pores to the entire volume of the porous component thereof) is at least 45%. Such a prosthesis is provided for the replacement of hard tissues of human bones or joints, or as a prosthesis which is partially composed of the above-mentioned porous lamination component at a desired surface portion of the prosthesis base. The term "base" here implies a portion to be embedded in the living bone tissue (hereinafter only referred to as "base") . Another aspect of the present invention provides a method of making such a prosthesis for the replacement of hard tissues of human bones and joints comprising the following steps of:
perforating metal thin sheets each having a thickness of 150 µm or less to provide a plurality of through holes, and being unharmful to the living body;
laying said metal thin sheets thus obtained over one another so that said through holes may communicate with one another in the direction of the thickness thereof; and
diffusion-bonding between said metal thin sheets into one body by heating so as to form a porous lamination component for the prosthesis whereby a prosthesis having a volume porosity of at least 45% is obtainable.

### BRIEF DESCRIPTION OF THE DRAWINGS

Fig 1 is a perspective view of a porous lamination component of an example of the present invention.
Fig. 2 is a plan view illustrating the arrangement of through holes in the thin sheets of the porous lamination component shown in Fig. 1.
Fig. 3 is a view taken on line I-I of Fig. 1,
Fig. 4 is a vertical sectional view of the porous lamination component of another example of the present invention.
Fig. 5 is a plan view illustrating a flat surface shape of a thin sheet of the present invention.
Fig. 6 is a plan view illustrating another flat surface shape on a thin sheet of the present invention.
Fig. 7 is a plan view illustrating still another flat surface shape of a thin sheet of the present invention.
Fig. 8 is a vertical sectional view of a porous lamination component on the present invention, illustrating a lamination condition of the through holes.
Fig. 9 is a view similar to Fig. 8, illustrating another lamination condition on the through holes.
Fig. 10 is a view similar to Fig. 8, illustrating still another lamination condition of the through holes.
Fig. 11 is a schema illustrating an animal experiment using the porous lamination component shown in Fig. 3.
Fig. 12 is a view similar to Fig. 11, illustrating another animal experiment.
Fig. 13 is a view similar to Fig. 11, illustrating still another animal experiment
Fig. 14 is a graph illustrating the measurement results of the adhesion strength of a porous lamination component of the present invention.
Fig. 15 is a microphotograph illustrating a histopathological examination result of a porous lamination component of the present invention used for an animal experiment.
Fig. 16 is a microphotograph illustrating another histopathological examination result of a porous lamination component of the present invention used for an animal experiment.
Fig. 17 is a microphotograph illustrating still another histopathological examination result of a porous lamination component of the present invention used for an animal experiment.
Fig. 18 is a perspective view illustrating an artificial vertebral body of the present invention.
Fig. 19 is a side view illustrating a condition wherein the artificial vertebral body shown in Fig. 18 is inserted between intervertebral disks.
Fig. 20 is a perspective view illustrating an artificial dental root of the present invention.
Fig. 21 is a top view illustrating the band-shaped thin sheet shown in Fig. 21.
Fig. 22 is a sectional view illustrating a method of laminating the porous lamination component for the artificial denial root shown in Fig. 21.
Fig. 23 is a side view illustrating the main body of the artificial dental root shown in Fig. 21.
Fig. 24 is a perspective view illustrating a femoral prosthesis of the present invention.
Fig. 25 is a side view of an artificial hip joint of the present invention.
Fig. 26 is a plan view illustrating the thin sheet used to form an acetabular porous lamination component on the present invention.
Fig. 27 is a side view of the acetabular shell body shown in Fig. 26.
Fig. 28 is a view illustrating a condition wherein an acetabular porous lamination component shown in Fig. 26 is formed.
Fig. 29 is a sectional view taken on line II-II of Fig. 28.
Fig. 30 is a side view of a femoral stem of the present invention.
Fig. 31 is a sectional view taken on line III-III on Fig. 30.
Fig. 32 is a plan view illustrating a thin sheet composing the porous lamination component for a femoral stem of the present invention.
Fig. 33 is a view illustrating a condition wherein the porous lamination component for a femoral stem of the present invention is formed.
Fig. 34 is a perspective view illustrating a femoral head cup on the present invention.
Fig. 35 is a sectional view taken on line IV-IV on Fig. 34.
Fig. 36 is a perspective view illustrating a condition wherein a femoral bone is replaced with a femoral head cup of the present invention.
Fig. 37 is a perspective view illustrating an artificial vertebral body of the present invention.
Fig. 38 is a top view of an artificial vertebral body of the present invention, and
Fig. 39 is a sectional view taken on line V-V of Fig. 38.

### DETAILED DESCRIPTION OF THE INVENTION

According to the present invention, porous thin sheets 1, 2,..., each having a thickness of 150 µm or less, are laminated and formed in a desired prosthesis shape, or laminated on the flat or curved surface of a prosthesis base. In the thin sheets 1, 2,..., small through holes H having a shape suited nor the penetration and ingrowth of a plurality of bone cells are provided. In addition, a prosthesis surface having a porous lamination component S1 with a three-dimensional cubic structure is formed by slightly displacing the positions of the through holes disposed in laminated thin sheets in the direction of the depth. The bone tissues penetrated and grown in the three-dimensional space inside the porous surface functions to firmly support the prosthesis in the living body by utilizing its cubic structure, thereby preventing the micro-movement of the prosthesis. Furthermore, by coating a bio-compatible material, in which bone cells can easily grow, on the laminated thin sheets, the penetration of bone cells can be promoted more promptly after the replacement operation of the prosthesis, thereby permitting earlier fixture to the bone to be joined. Moreover, when a prosthesis made by laminating the thin sheets 1, 2,... having through holes H with an effective diameter of 100 to 400 µm is secured with bone cement, the fixture performance between the bone cement and the prosthesis can be enhanced significantly.

### EXAMPLES

Referring to the accompanying drawings, the examples of the present invention will be explained specifically.

### (Example 1)

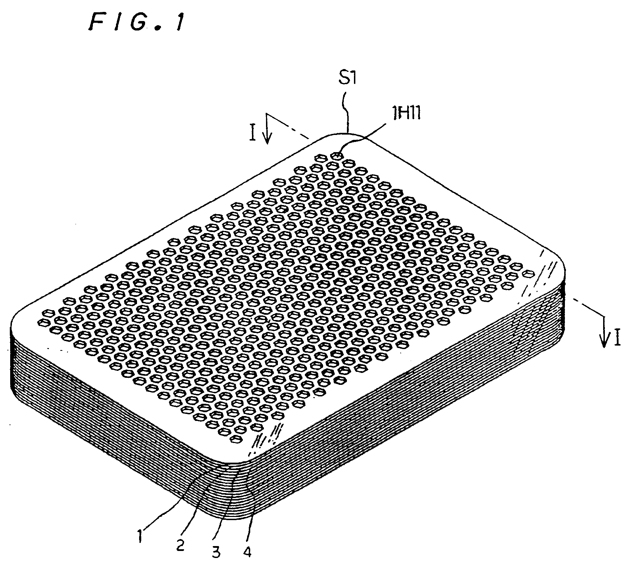

Fig. 1 is a perspective view of a porous lamination component S1 comprising the whole of a prosthesis, or a part or the whole of the base surface of a prosthesis of the present invention. The porous lamination component S1 measures 10 x 15 x 2 mm. It has a sequentially laminated structure including 20 layers of porous thin sheets 1, 2,..., each having a thickness of 100 µm and a plurality of through holes. The thin sheets 1, 2,... are made of pure titanium. After the thin sheets 1, 2,... were positioned properly while being laminated, and secured temporarily by placing a very light weight on them or by using adhesive, they were heated at about 900°C in a vacuum sintering furnace so that they were bonded mutually. The heater of the sintering furnace is made on molybdenum. The heating process can be conducted in an atmosphere of inert gas such as argon. The bonding on the thin sheets 1, 2,... was conducted by diffusion-bonding between metal atoms. The thin sheets 1, 2,... were positioned by utilizing the rectangular sides thereof as reference sides. In the process inside the vacuum furnace, although the overlap on the through holes H may be dislocated slightly, the amount on the dislocation is about 20 µm and is almost negligible nor proper production. If more precise overlap is necessary, or in the external shape of the porous lamination component S1 has a shape other than a cube and has not any flat surface portions which can be used as reference surfaces, positioning holes (not shown) should preferably be provided at the four corners of the thin sheets 1, 2,... After the thin sheets 1, 2,... were diffusion-bonded mutually in this way, no special change was recognized in the appearance and dimensions on the porous lamination component S1 and no deteriorated layer was recognized.

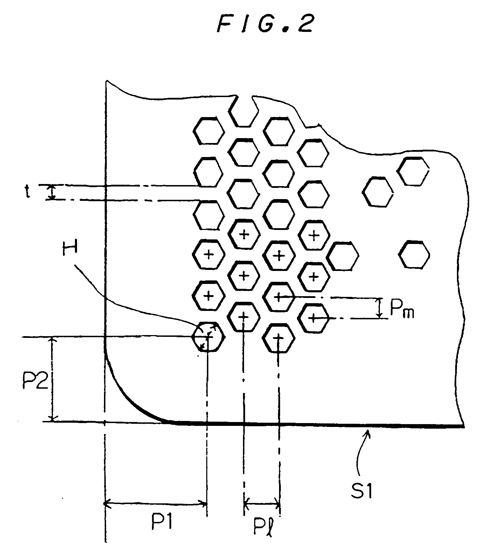

Fig. 2 shows a magnified view of regular hexagonal through holes H formed in the thin sheets 1, 2, 3, 4,... of the above-mentioned porous lamination component S1. The shape of the regular hexagonal through holes H is one of shapes suited for easy penetration and dense ingrowth of osteon, the minimum unit of bone tissues. The shape is also suited for allowing the highest density filling arrangement on the through holes.in the same way as that shown in honeycombs and crystal structures. As the dimension on a pore which permits bone tissues to penetrate easily, the diameter of the inscribed circle on the through hole H of this example was determined to be about 350 µm. Since the etching method as described below was used to form the through holes H in this example, the central sectional portion of the through hole H was raised. The diameter of the inscribed circle at the mostly raised portion became about 300 µm. The porous lamination component S1 was designed as shown in Fig. 2; the horizontal width of the non-pore fringe portion P1 = 1.05 mm, the vertical width of the non-pore fringe portion P2 = 1.27 mm, the horizontal interval between the through holes Pl = 500 µm, and the vertical interval between the through holes Pm = 433 µm, thereby most densely arranging the through holes H with an effective diameter of 350 µm. The space t between the through holes H resulted in 150 µm. The volume porosity of the pores in the porous lamination component S1 designed in this way is about 50% per unit volume, that is, the volume porosity of the porous lamination component S1 is 50%. In this way, the volume porosity of the porous lamination component S1 can be easily controlled by appropriately adjusting the effective diameter of the through holes H, the interval between the through holes H and the combination of lamination layers. For example, in Pl = 450 µm and Pm = 383 µm in the above example, the volume porosity can be changed to about 60%. Furthermore, if Pl = 450 µm, Pm = 383 µm and the effective diameter of the through holes H is 400 µm in the above example, the volume porosity can be changed to about 75%. The volume porosity should preferably be as large as possible to make the amount of bone tissues to penetrate as much as possible.

The above-mentioned through holes H were formed by an etching method. The through holes H can also be formed by laser processing or punching.

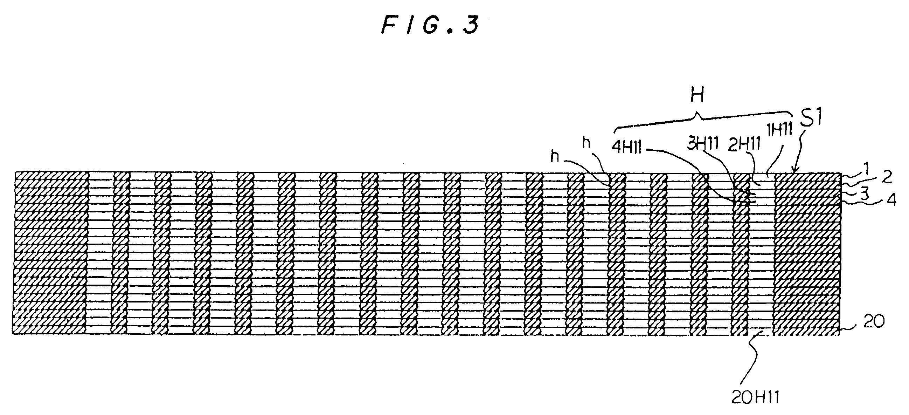

Fig. 3 is a sectional view of the porous lamination component S1 taken on line I-I of Fig. 1. To design the distribution of the through holes H, the through hole H disposed at the n-th line and the m-th column of the first layer is defined to be represented as 1Hnm. The through hole H represented by 1H11 is a through hole disposed at the first line and the first column of the first layer. The thin sheets 1, 2,... with a thickness of 100 µm are laminated and the through holes 1H11 and 2H11 communicate with each other. In actual practice, however, the layers of the porous lamination component S1 are often dislocated slightly as described above. When the thin sheet is corroded on both sides thereof in the etching liquid showering process to form through holes H, the cross section of the sheet has a shape similar to a rhombus because of the projection section h projecting toward the center of the through hole H as shown in the figure, and the angle of the inclined surface thereof is in the range of about 30 to 45 degrees. If the thin sheet is etched on the one side thereof, the cross section of the sheet has a shape similar to an isosceles triangle. Whether the thin sheet is etched on both sides or on one side is determined by considering the shape of the prosthesis to be used and the biomechanical conditions at the hard tissue portion to be replaced. More particularly, it is necessary to consider what kind of stress and how much stress remains on the surface of the porous lamination component S1. In addition, when bone cement is used for fixture, the porous lamination component S1 should be designed so that bone cement can easily penetrate the through holes H and cannot come out easily after polymerization.

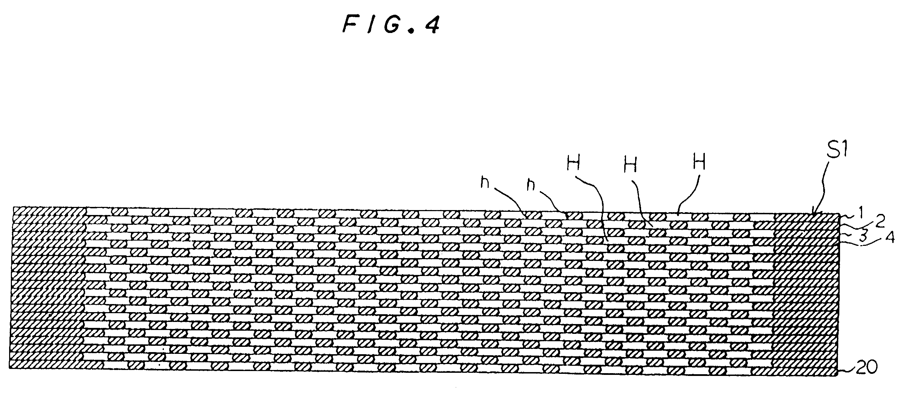

The porous lamination component S1 processed as described above has slight dislocations at laminated portions. For example, the positions of the through holes 1H11 and 2H11 are dislocated slightly from their designed positions. If the through holes H in the thin sheets 1, 2,... are designed to be disposed alternately as shown in Fig. 4, more spaces can be provided to guide bone tissues. With this structure, bone tissues can penetrate the prosthesis for an extended period of time, thereby being more effective for the support of the prosthesis. This kind of uniform plan view shape and three-dimensional structure of the through holes H can be formed on any prosthesis surfaces by utilizing the present invention. The plan view shape of the through holes H should preferably be a regular hexagonal shape having the highest density filling efficiency, and should also be a shape capable of increasing the volume porosity as large as possible. In addition, the through holes H adjacent to one another should preferably have open three-dimensional structures contacting one another. Furthermore, to shorten the period requiring nor permitting bone tissues to penetrate, the through holes H should preferably be coated with a bio-active living body material having affinity to bone tissues. As the material used for coating, bioglass ceramics, chitin, chitosan and gelatin can be nominated. In particular, when no bio-active living body material is coated, titanium oxide coating or titanium nitride coating superior in corrosion resistance in the living body should preferably be used to prevent metal ions from flowing out. Moreover, when bone cement is used to secure the prosthesis, the through holes H should preferably be coated with an agent, such as a silane coupling agent, which can strengthen the force of adhesion to bone cement.

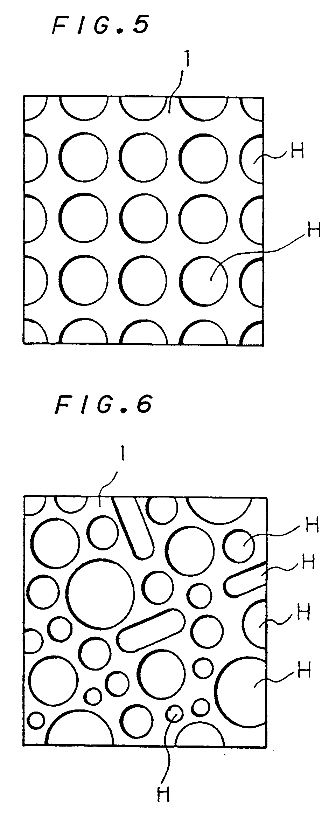

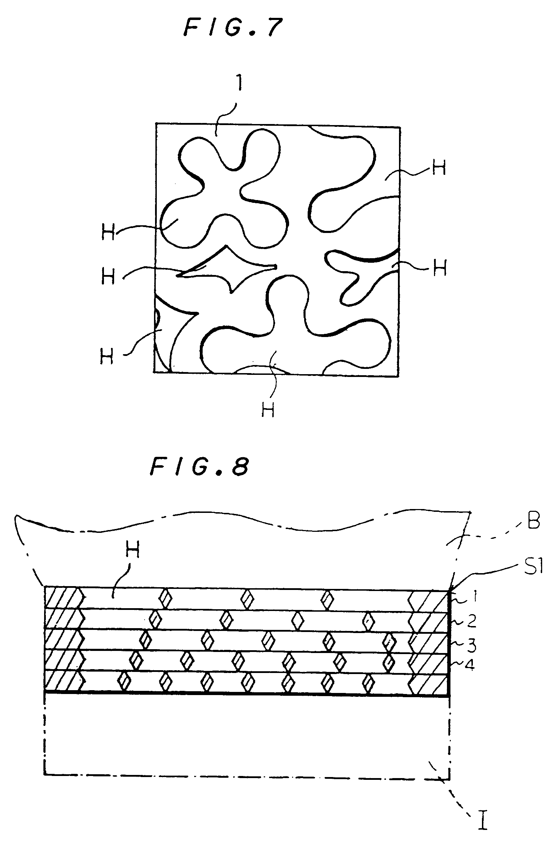

Figs. 5, 6 and 7 show various plan view shapes of the through holes H. The plan view shapes of the through holes H should be suited for the penetration of bone cells. Fig. 5 shows the through holes H disposed at the highest density arrangement. Fig. 6 shows the through holes H with various diameters. Although it is generally said that pores with an inscribed circle diameter on 75 to 350 µm are suited for promoting the penetration of bone cells, a plan view shape having distributed pores with various diameters in a constant area, such as that shown in Fig. 6, can also be considered to be effective. This kind of plan view shape can also be obtained easily by utilizing the present invention. In some cases, indeterminate forms of through holes H shown in Fig. 7 may be desirable. Depending on the kind of bone cells, a proper response may not necessarily be obtained by circular through holes, but a tapered shape may be preferable.

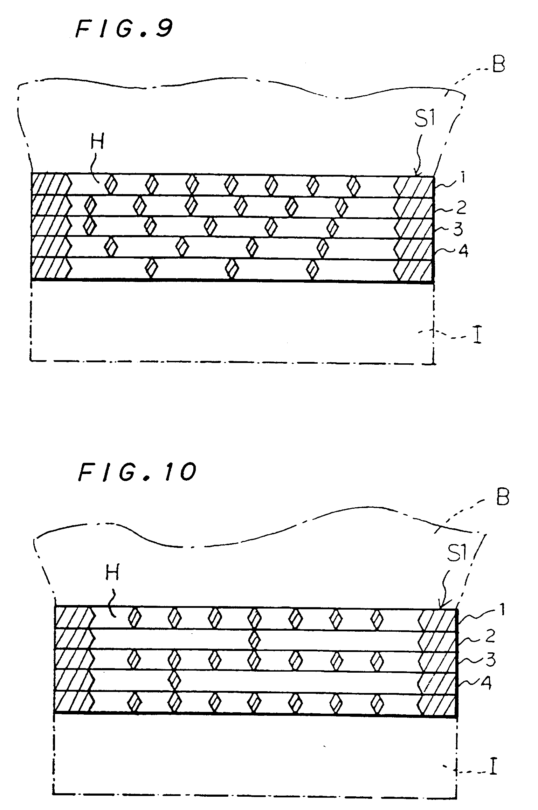

Figs. 8, 9 and 10 show the sectional structures on the through holes H in the laminated thin sheets 1, 2,... Fig. 8 shows a sectional structure wherein the effective dimensions of the through holes H disposed in the thin sheets 1, 2,... become smaller as the through holes H are disposed closer to the prosthesis base I from the bone tissue side B. Fig. 9 shows a sectional structure which is opposite to that shown in Fig. 8. This sectional structure is particularly effective when a tension load is applied to the porous lamination component surface. Fig. 10 shows a sectional structure wherein the areas on the through holes H in the second and fourth layers are especially larger than those of the through holes in the other layers. When the shape of the prosthesis base I is curved and the curvature of the shape is small, the positional relationship among the thin sheets are dislocated slightly. Although it is possible to design the thin sheets 1, 2,... by considering the effect of the slight dislocation previously estimated, the dislocation can be neglected to some extent by using the structure shown in Fig. 10. In addition, it is also said that the structure wherein the diameters of the through holes H are made larger in the upward or downward direction is suited for the ingrowth of bone tissues.

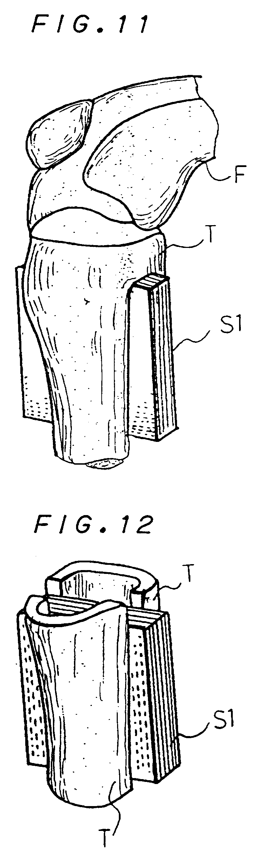

Fig. 11 shows a schema illustrating the porous lamination component S1 made as described above and used for an animal experiment to examine the effectiveness thereof. The porous lamination component S1 used for this experiment has through holes H which pass through straight in the vertical direction as shown in Fig. 3. The effective diameter of the through hole H is 350 µm and the volume porosity of the porous lamination component S1 is 60%. The experiment was conducted in accordance with the method described in the Journal of Biomedical Materials Research (hereinafter referred to as "JBMR"), Vol. 20, 1295-1307 (1986). The porous lamination component S1 was embedded at a position about 3 cm from a mesial tibia T of rabbits. The porous lamination component S1 was cleansed, autoclaved, and wetted with a saline solution including a dissolved antibiotic. The porous lamination component S1 was then embedded into a gutter by pounding it with a hammer. The periosteum, muscle, fascia and skin were stitched up together in accordance with the conventional methods. After the antibiotic was given, the treatment portion was laid quietly. Each rabbit was allowed to move freely in a cage (50 x 80 x 40 cm) and was fed with solid food and water.

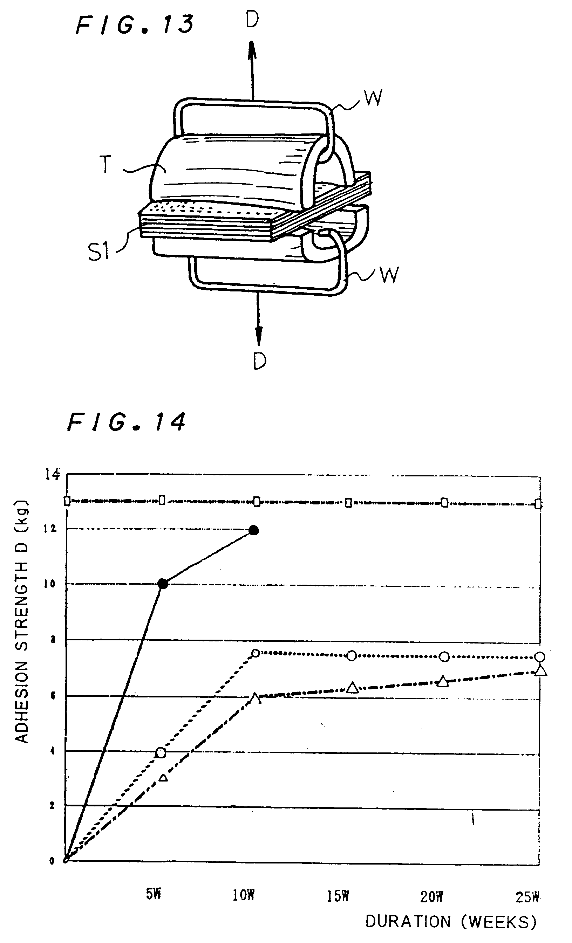

The rabbits were killed four and six weeks after operation. As shown in Fig. 12, the porous lamination component S1 and a part of the tibia T around the porous lamination component were taken out. After about two hours, without formalin fixation, a wire W was passed through the tibia T as shown in Fig. 13 and the adhesion strength D of the structure was measured by using an instron testing machine. The loading condition of the instron testing machine was given at a cross head speed of 3.5 cm/min in accordance with the above-mentioned method.

Fig. 14 is a graph illustrating the measurement results of the adhesion strength D. The points marked ● indicate the adhesion strength of the porous lamination component S1 of the present invention. The adhesion strength of the porous lamination component S1 was about 118 Nw (10 kg) five weeks after adhesion. After ten weeks, the adhesion strength of the S1 was about 98 Nw (12 kg). The adhesion strength per unit area was 588 KPa (6 kg/cm²) after five weeks and 785 KPa (8 kg/cm²) after ten weeks. Breakage occurred in the tibia T attached to the porous lamination component S1. No separation was observed at the interfaces of the thin sheets of the porous lamination component S1. The points marked o indicate the adhesion strength of the bioglass ceramics reported in the above-mentioned literature. It was reported that the average adhesion strength was 74,63 Nw (7.61 kg) after ten weeks and 71,00 Nw (7.24 kg) after 25 weeks. As reported in JBMR, Vol. 23, 781-808 (1989), the average adhesion strength of hydroxyapatite was 62,76 Nw (6.40 kg) after eight weeks and 67,27 Nw (6.86 kg) after 25 weeks as indicated by the points marked Δ. The points marked □ indicate the strength of the bone. It was reported that the average adhesion strength was 117,29 Nw (11.96) kg after 25 weeks according to JBMR, Vol. 19, 685-698 (1985). Since these experiments were conducted in the same conditions, the values of the measurement results can be used for comparison. According to these results, it is found that the porous lamination component S1 of the present invention is superior to the bioglass ceramics and hydroxyapatite, which are assumed to be the most bio-active materials among the currently available living body materials in terms of the performance of bonding and adhesion to bone tissues. It is also found that the adhesion strength of the prosthesis reaches a value almost similar to the strength of the bone in a short period of about ten weeks.

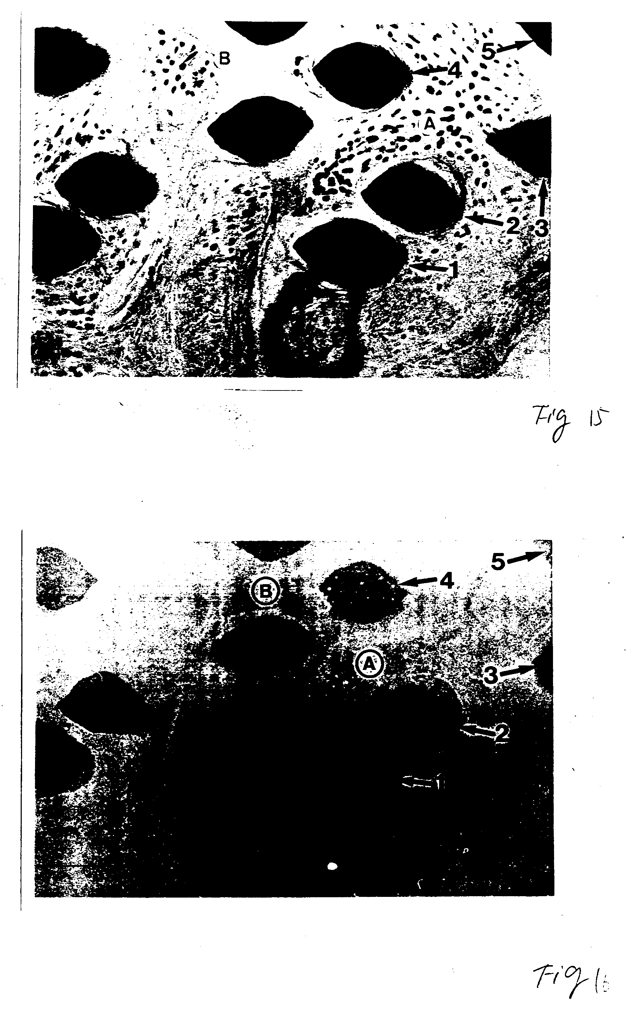

Figs. 15, 16 and 17 are microphotographs illustrating the histopathological examination results of the porous lamination component S1 used for the adhesion strength measurement tests.

The bone tissues obtained after the adhesion strength tests and the porous lamination component S1 were subjected to dehydration and resin embedding in accordance with the conventional methods, and sliced in the direction perpendicular to the longitudinal axis of the tibia T, then manually processed to obtain ground sections Z with a thickness of up to about 50 µm. The sections were observed by using a light microscope in accordance with the normal light microscopy method and the fluorescent microscopy method.

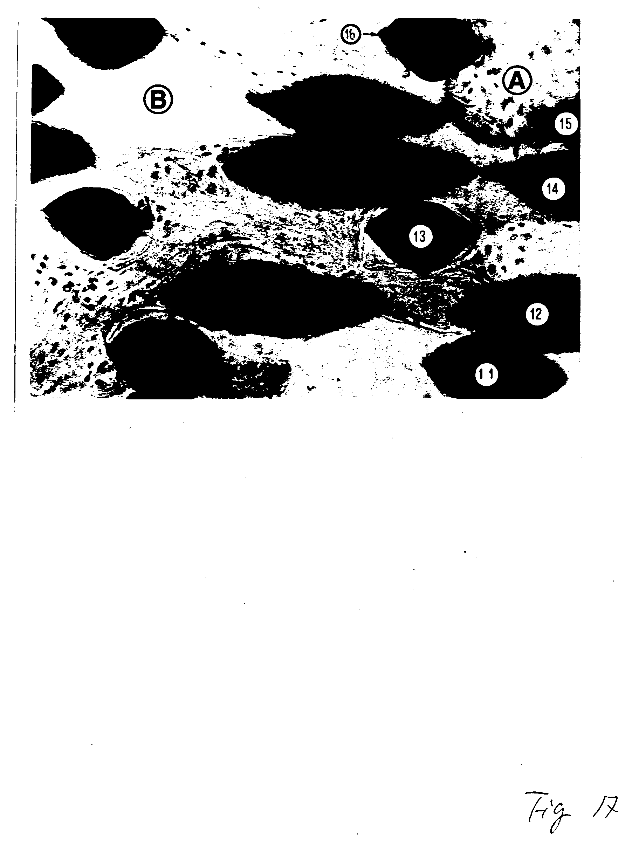

Fig. 15 is a microphotograph at 5500 magnifications. It is found that bone tissue A and marrow tissue B coexist in the through holes H in the regions of the thin sheets 1 to 5. In addition, blood vessel tissues and new osteoblasts were observed with the naked eye, although they were not taken clearly on the microphotograph. Fig. 16 shows a fluorescent microscopic image of the image shown in Fig. 15.

Fig. 17 shows the regions corresponding to the thin sheets 11 to 15. Although the amount of the bone tissue A is smaller, there are no great differences in the calcification degree and the adhesion strength of the bone tissue.

As shown in Figs. 15 to 17, the bone tissue A penetrated almost all through holes H of the thin sheets 1 to 20 of the porous lamination component S1 grew in the through holes. It is found that the tissue A is a normal bone tissue including marrow tissue B. This result indicates that a complex substance is formed between the surface of the porous lamination component of the present invention and the bone and that the metallic thin sheets reinforce the bone tissues. In addition, it is found that the bonding strength of the porous lamination component S1 is sufficient and that the force of adhesion to the bone is almost equal to the breakage force of the bone itself.

Next, other samples of the porous lamination component S1 comprising thin sheets 1, 2,... having different thickness values listed in Table 1 (different from the thickness of the thin sheets on the porous lamination component S1 used for the above-mentioned experiments) were made in accordance with the above-mentioned method. These samples of the porous lamination component S1 were used for the same animal experiments as those described above and the adhesion strength values were measured after ten weeks. The measurement results are shown in Table 1.

**[Table 1]**

| Sample No. | Thickness of thin sheet (µm) | Adhesion strength KPa (kg/cm²) |
|---|---|---|
| 1 | 5 | 892 (9.1) |
| 2 | 10 | 1177 (12) |
| 3 | 50 | 1177 (12) |
| 4 | 100 | 1177 (12) |
| 5 | 150 | 1177 (12) |
| 6 | 175 | 814 (8.3) |

As obviously indicated by Table 1, when the above-mentioned thickness is in the range of 10 to 150 µm, the adhesion strength is 1177 KPa (12 kg/cm²). This value is almost equal to the strength of the bone as described above. It was confirmed that a breakage occurred at the tibia T. On the other hand, when the thickness is larger than 150 µm, the adhesion strength is less than 981 KPa (10 kg/cm²). In this case, it was also confirmed that a breakage occurred in the porous lamination component S1.

This breakage occurrence is assumably explained as follows. When the above-mentioned thickness is in the range of 10 to 150 µm, the bone cells which penetrate the through holes H and grow therein are firmly anchored by the projection portions h of the thin sheets 1, 2,... projecting in the central direction of the through holes H as shown in the sectional view of Fig. 3. When the thickness is smaller than 10 µm, the amount of the projection at the projection portions h is too small and the bone cells are not sufficiently supported, thereby being incapable of firmly anchoring the bone cells. When the thickness is larger than 150 µm, the amount of the projection at the projection portions h is large, and the diameter of the through holes H in the direction of the thickness is reduced to about 10 µm, thereby assumably resulting in the breakage of the bone tissues at the region of the reduced diameter portions.

Furthermore, when the thickness is smaller than 10 µm, the number of the thin sheets 1, 2,... to be laminated increases significantly. This makes the production of the porous lamination component extremely difficult.

Next, still other samples of the porous lamination component S1 comprising thin sheets 1, 2,... having the through holes alternately disposed as shown in Fig. 4 and also having different thickness values listed in Table 2 were made in accordance with the above-mentioned method. The shape of the through holes H in the porous lamination component S1 was hexagonal and its effective diameter was 300 µm and the volume porosity of the porous lamination component S1 was 60%. These samples of the porous lamination component S1 were subjected to the above-mentioned animal experiments and the adhesion strength values were measured after ten weeks. The measurement results are shown in Table 2.

**[Table 2]**

| Sample No. | Thickness of thin sheet (µm) | Adhesion strength KPa (kg/cm²) |
|---|---|---|
| 7 | 5 | 932 (9.5) |
| 8 | 10 | 1177 (12) |
| 9 | 50 | 1177 (12) |
| 10 | 100 | 1177 (12) |
| 11 | 150 | 1177 (12) |
| 12 | 175 | 971 (9.9) |

As obviously indicated by Table 2, when the above-mentioned thickness is in the range of 10 to 150 µm, the adhesion strength is 1177 KPa (12 kg/cm²). This value is almost equal to the strength of the bone as described above. It was confirmed that a breakage occurred at the tibia T. On the other hand, when the thickness is smaller than 10 µm or larger than 150 µm, the adhesion strength is less than 981 KPa (10 kg/cm²). In this case, it was confirmed that a breakage occurred in the porous lamination component S1.

Next, still other samples of the porous lamination component S1 comprising thin sheets 1, 2,... having a thickness of 100 µm and also having the through holes H with different effective diameters listed in Table 3 were made in accordance with the above-mentioned method. The cross sectional structure of this porous lamination component S1 had the through holes H alternately disposed in the thin sheets 1, 2,... as shown in Fig. 4 and the volume porosity of the porous lamination component S1 was 60%. These samples of the porous lamination component S1 were subjected to the above-mentioned animal experiments and the adhesion strength values were measured after ten weeks. The measurement results are shown in Table 3.

**[Table 3]**

| Sample Effective hole diameter No. (µm) | | Adhesion strength KPa (kg/cm²) |
|---|---|---|
| 13 | 50 | 706 (7.2) |
| 14 | 75 | 853 (8.7) |
| 15 | 100 | 1177 (12) |
| 16 | 200 | 1177 (12) |
| 17 | 300 | 1177 (12) |
| 18 | 400 | 1177 (12) |
| 19 | 500 | 912 (9.3) |

As obviously indicated by Table 3, when the above-mentioned effective hole diameter is in the range of 100 to 400 µm, the adhesion strength is 1177 KPa (12 kg/cm²). This value is almost equal to the strength of the bone as described above. It was confirmed that a breakage occurred at the tibia T. On the other hand, when the diameter is smaller than 100 µm or larger than 400 µm, the adhesion strength is less than 981 KPa (10 kg/cm²). In this case, it was confirmed that a breakage occurred in the porous lamination component S1.

Next, yet still other samples of the porous lamination component S1 comprising thin sheets 1, 2,... having a thickness of 100 µm and the through holes H with an effective diameter of 300 µm and also having different volume porosity values listed in Table 4 were made in accordance with the above-mentioned method. The cross sectional structure of the porous lamination component S1 had the through holes H alternately disposed in the thin sheets 1, 2,... as shown in Fig. 4.

**[Table 4]**

| Sample No. | Volume porosity (%) | Adhesion strength KPa (kg/cm²) |
|---|---|---|
| 20 | 40 | 961 (9.8) |
| 21 | 45 | 1177 (12) |
| 22 | 50 | 1177 (12) |
| 23 | 60 | 1177 (12) |
| 24 | 70 | 1177 (12) |

As obviously indicated by Table 4, when the above-mentioned volume porosity is more than 45%, the adhesion strength is 1177 KPa (12 kg/cm²). This value is almost equal to the strength of the bone as described above. It was confirmed that a breakage occurred at the tibia T. On the other hand, when the volume porosity is less than 45%, the adhesion strength is less than 981 KPa (10 kg/cm²). In this case, it was confirmed that a breakage occurred in the porous lamination component S1.

Accordingly, it is found that the preferable thickness of the thin sheets 1, 2,... is 150 µm or less, more particularly in the range of 10 to 150 µm, the preferable effective hole diameter is in the range of 100 to 400 µm and the preferable volume porosity of the porous lamination component S1 is 45% or more.

### (Example 2)

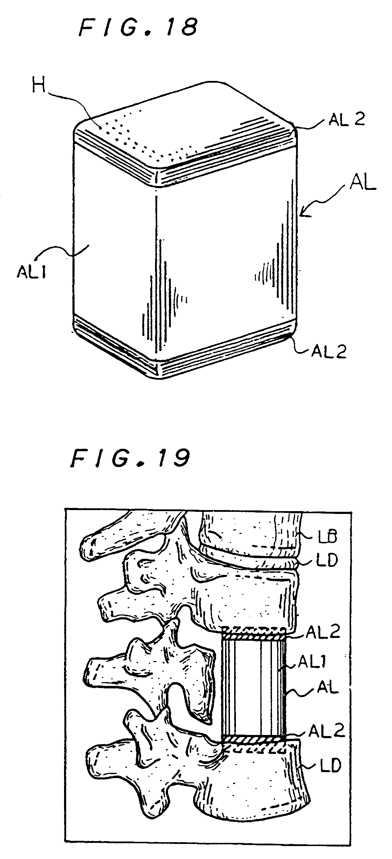

Fig. 18 shows an artificial vertebral body AL used as a prosthesis of an example on the present invention. Ten pieces of 100 µm thick titanium thin sheets 1, 2,... having the same shape as that on the end surface of the artificial vertebral body AL were laminated to form a porous lamination component AL2 having a thickness of about 1 mm and this porous lamination component AL2 was bonded to both ends of the cubic artificial vertebral body AL made of a titanium alloy. The effective diameter of the through holes H was 300 µm. Other design items were the same as those of the example 1.

The artificial vertebral main body AL1 laminated with the porous lamination component AL2 comprising the lamination of the above-mentioned thin sheets 1, 2,... as shown in Fig. 18 was heated up to about 900°C in a vacuum furnace in an atmosphere of inert gas (argon). The surfaces of the artificial vertebral body AL were then coated with hydroxyapatite by flame spray coating. Next, the hydroxyapatite was converted into a paste state, and recrystallized by heat treatment. Bioglass ceramics was then used for coating.

In addition, to coat hydroxyapatite on the internal wall surfaces of the through holes H of the above-mentioned porous lamination component AL2, an artificial vertebral body AL was made as described below.

First, both ends of the artificial vertebral main body AL1 were coated with hydroxyapatite, and the porous lamination components AL2 were laminated on the artificial vertebral main body AL1 and then these laminated components were heated in the vacuum furnace as described above. Hydroxyapatite was coated only on the internal wall surfaces of the through holes H disposed close to the interfaces between the porous lamination components AL2 and the artificial vertebral main body AL1.

Furthermore, hydroxyapatite coating was performed by flame spray coating on one end of the porous lamination component AL2 which had been made previously in accordance with the method used for example 1. By heating the spray-coated surface abutted to the artificial vertebral main body AL1 in the vacuum furnace, an artificial vertebral body AL, in which the internal wall surfaces of the through holes H were coated with hydroxyapatite only on the intermediate layers of the porous lamination component AL2, was obtained.

In this way, the coating range in the cross section of the porous lamination component can be controlled.

Fig. 19 is a side view illustrating the condition wherein a lumbar vertebra LB and an intervertebral disk LD are replaced with the above-mentioned artificial vertebral body AL.

In addition to the artificial vertebral body, this example can be applied to various prostheses such as spinous process spacers, iliac bone spacers, posterior cranial fossa plates and artificial knee joint tibial components.

### (Example 3)

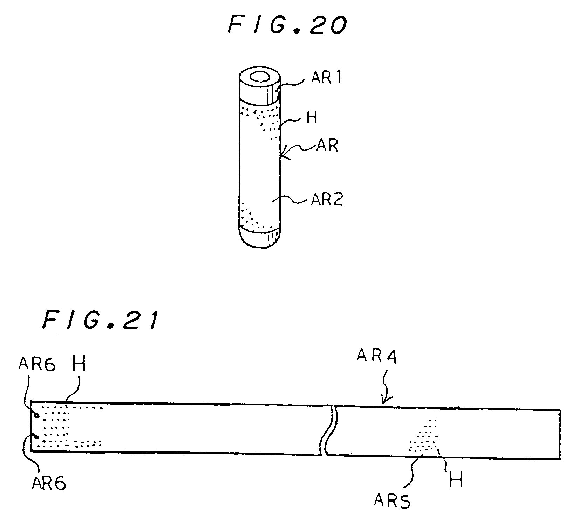

Fig. 20 is a perspective view illustrating an artificial dental root AR of the present invention. The artificial dental root AR comprises a cylindrical artificial dental root body AR1 and an artificial dental root porous lamination component AR2 formed around the cylindrical surface of the artificial dental root body AR1.

Fig. 21 is a plan view of a thin sheet AR4 which is used to form the artificial dental root porous lamination component AR2. The thin sheet AR4 has a shape of band measuring 50 µm in thickness, 7.9 mm in width and 1000 mm in length. It has a plurality of through holes H with an effective diameter of about 300 µm, with non-pore fringe portions AR5 having a horizontal width of about 1 mm provided around the external fringe of the band. Furthermore, at the leading end of the above-mentioned thin sheet AR4, two fixture holes AR6 are provided.

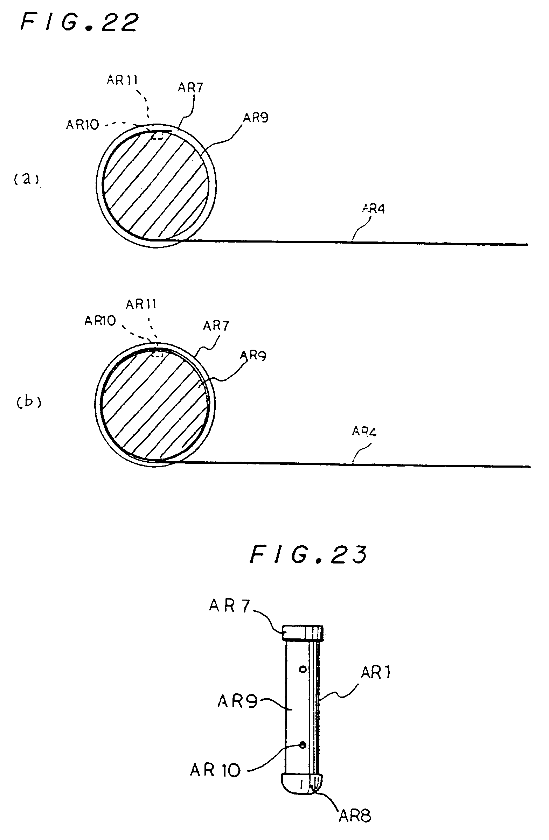

Fig. 22 is a sectional view illustrating a method of laminating the band-shaped artificial dental root porous lamination component AR2 over the porous lamination component accommodation section AR9 of the artificial dental root body AR1. The artificial dental root body AR1 was secured at the axial center thereof. Two 0.85 mm diameter titanium rods AR11 were respectively driven into the two thin sheet fixture holes AR10 disposed in the porous lamination component accommodation section AR9 of the artificial dental root body AR1 shown in Fig. 23. The above-mentioned fixture holes AR6 disposed in the thin sheet AR4 were fitted over the rods AR11. The projected portions of the rods AR11 were then removed by filing operation. After this, while the thin sheet AR4 was pulled to prevent it from being deflected, the artificial dental root body AR1 was rotated to wind the thin sheet AR4 around the artificial dental root body AR1.

Since the thickness of the thin sheet AR4 was 50 µm, the thickness of the artificial dental root porous lamination component AR2 was equal to the depth of the above-mentioned porous lamination component accommodation section, i. e., 1 mm after the thin sheet AR4 was rotated about 20 times. The thin sheet AR4 was then cut at an appropriate position, the trailing end thereof was temporarily secured by using adhesive and heated at about 900°C in the vacuum furnace.

Fig. 23 is a side view of the artificial dental root body AR1. The artificial dental root body AR1 is a cylinder with an overall length of 12 mm and comprises a 4 mm diameter end section AR7 disposed at one end thereof, a leading end section AR6 disposed at the other end thereof and having a round end, and the porous lamination component accommodation section AR9 disposed between the end section AR7 and the leading end section AR6 and having a length of about 8 mm and a diameter of 3 mm. The porous lamination component accommodation section AR9 is provided with two thin sheet fixture holes AR10 with a diameter of 0.8 mm in the porous lamination component accommodation section AR9.

It was possible to make the artificial dental root AR comprising the porous lamination component AR2 in the same way as described above by using the above-mentioned thin sheets AR4 having thickness values of 75, 100, 125 and 150 µm. When the thin sheet AR4 having a thickness of 175 µm was used, however, it had to be pulled strongly when it was wound around the circumference of the above-mentioned porous lamination component accommodation section AR9, resulting in breakage of the fixture holes AR6. To prevent this breakage, it was attempted to apply smaller tension force during the winding process. In this case, the thin sheet was not able to be wound accurately in accordance with the curvature of the porous lamination component accommodation section AR9. Even though an artificial dental root AR was obtained by winding the thin sheet AR4 in some way, only partial bonding was accomplished even when the artificial dental root AR was heated in the vacuum furnace. For this reason, no practical artificial dental root was able to be made.

According to the results of the actual practice, the thin sheets having thickness values in the range of 50 to 100 µm were able to be used most easily for the production of the artificial dental root AR.

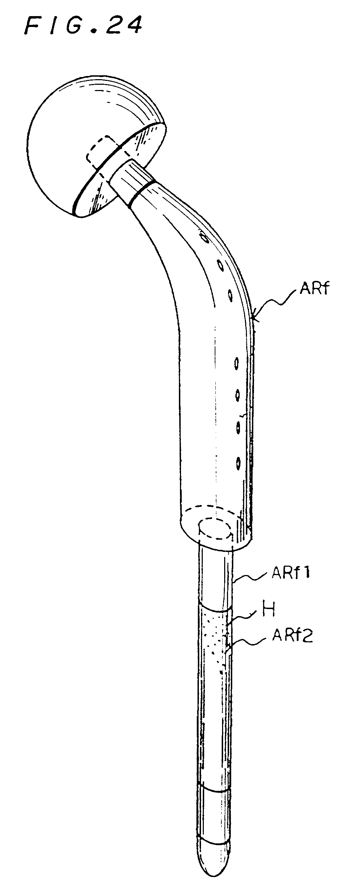

The femoral prosthesis ARf shown in Fig. 24 has a porous lamination component ARf2 around the circumference of a bone embedding section ARfl. This porous lamination component can be formed by the same method as that used to make the porous lamination component AR2 of the above-mentioned artificial dental root AR, wherein the process of winding the thin sheet AR4 is included.

### (Example 4)

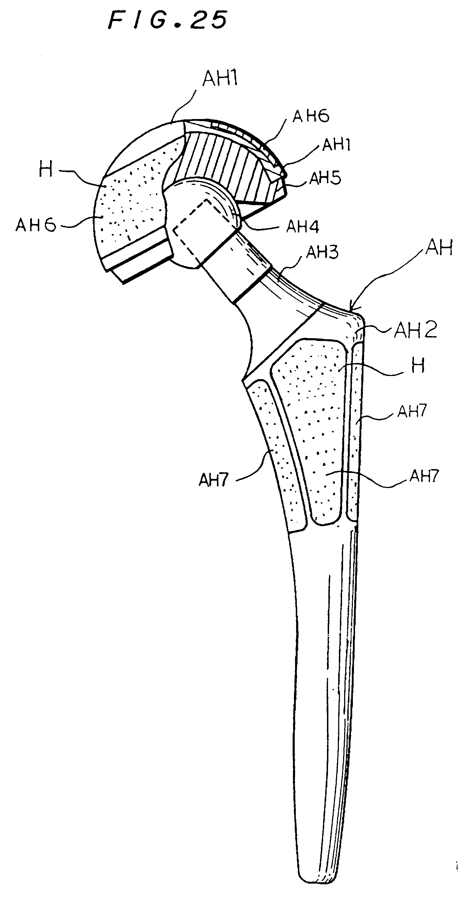

Fig. 25 shows an artificial hip joint AH. This artificial hip joint AH comprises a hemispherical acetabular shell body AH1 to be secured to a pelvis, a rod-shaped femoral stem AH2 to be inserted into a femoral medullary cavity, a hemispherical ball member AH4 to be fit in the leading end of a rod-shaped member AH3 extending at about 45 degrees from one end of the femoral stem AH2 and a bearing member AH5 used to form a ball joint in combination with the ball member AH4 and inserted in the interior of the acetabular shell body AH1.

An acetabular porous lamination component AH6 and a femoral stem porous lamination component AH7 are formed on the surfaces of the acetabular shell body AH1 and the femoral stem AH2. The acetabular shell body AH1 and the femoral stem AH2 are made of a titanium alloy, and the acetabular porous lamination component AH6 and the femoral stem porous lamination component AH7 are made of pure titanium.

The acetabular porous lamination component AH6 is disposed in the greater part of the region where it directly contacts the pelvis. The femoral stem porous lamination component AH7 is formed mainly around the entire circumference in the proximal region of the femoral medullary cavity.

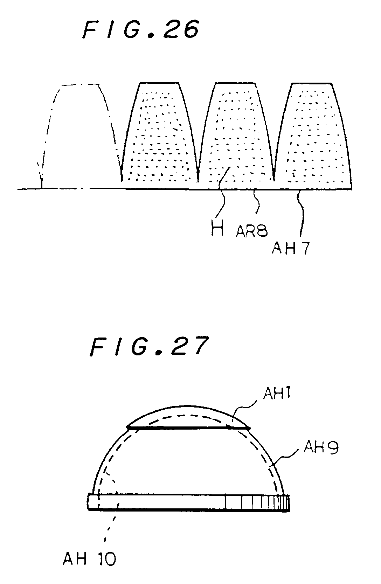

Fig. 26 is a plan view of the thin sheet AH7 used to form the acetabular lamination component AH6. The thin sheet AH7 with a thickness of 100 µm has a non-pore fringe portion AH8 with a width of 1 mm around the entire circumference thereof and is provided with a plurality of through holes H with an effective diameter of about 300 µm in the other sections. The thin sheet AH7 has a shape comprising several pieces of roughly isosceles triangular forms arranged continuously along the extension line of the base sides of the triangular forms, wherein each triangular form is made by respectively connecting the ends of the base side with the ends of the top side (shorter than the base side) of each fragmental section of the thin sheet using curved lines. The shape of the thin sheet AH7 is almost equal to the shape obtained by unfolding a spherical surface.

Fig. 27 is a side view illustrating only the above-mentioned acetabular shell body AH1. The acetabular shell body AH1 is roughly a hemisphere with a diameter of 50 mm. From the position 5 mm away from the end surface of the acetabular shell body AH1, an acetabular porous lamination component accommodation section AH9 having a spherical surface with a diameter of 48 mm is formed coaxially with the acetabular shell body AH1. The top end of the accommodation section AH9 is positioned 5 mm below the vertex of the acetabular shell body AH1. In the interior of the accommodation section AH9, an internal ball with a diameter of 45 mm is included, which contacts the bearing member AH5.

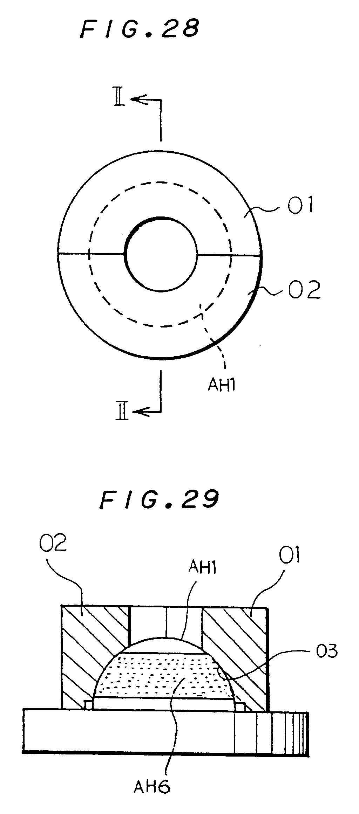

Fig. 28 shows a condition wherein the acetabular lamination component AH6 is formed on the acetabular porous lamination component accommodation section AH9 of the acetabular shell body AH1. The acetabular shell body AH1 is enclosed by metal molds O1 and O2. Fig. 29 is a sectional view taken on line II-II of Fig. 28. The metal molds O1 and O2 have an external shape obtained by dividing a cylinder measuring 70 mm in outer diameter and 50 mm in height into two pieces on the flat plane including the central axis of the cylinder. Inside the molds, a hemispheric bore O3 with a diameter of 50.5 mm is provided to allow the thin sheet AH7 to be formed around the entire circumference of the acetabular porous lamination component accommodation section AH9. When the acetabular shell body AH1 and ten pieces of the thin sheets AH7 are inserted in the metal molds O1 and O2 provided as described above, and the metal molds are made contact with each other at their division surfaces, the thin sheets AH7 are deformed into a hemispheric form inside the acetabular porous lamination component accommodation section AH9. To make the metal molds O1 and O2 contact with each other, screws can be used to pull the molds. The thin sheets AH6 can also be bent by pressing them against a cylindrical shaft with a diameter of 50 mm beforehand. By heating the thin sheets AH7 to about 900°C in the vacuum furnace, the acetabular porous lamination component accommodation section AH9 is bonded to the surface of the acetabular shell body AH1. To form a hemispherical porous lamination component, it is not always necessary to use hemispherical surfaces such as those provided in the metal molds O1 and O2, the object for obtaining a hemispherical surface can be attained by partially supporting the thin sheets at about three points.

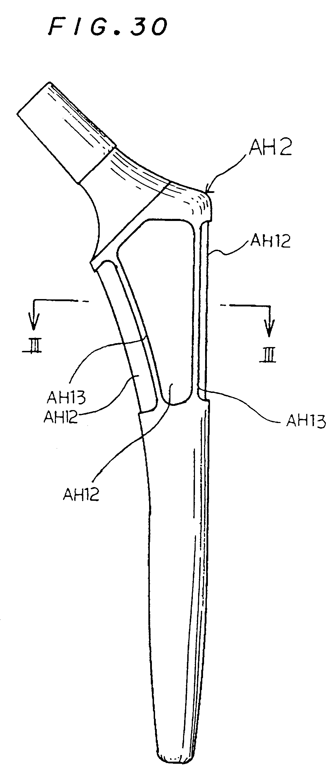

Fig. 30 is a side view of the femoral stem AH2. In the middle section of the femoral stem AH2, a femoral stem porous lamination component accommodation section AH12 is provided around the entire circumference of the stem AH2 in a width of about 60 mm. The femoral stem porous lamination component accommodation section AH12 comprises two flat surfaces and two curved surfaces, and these surfaces are partitioned by bank-shaped projections AH13.

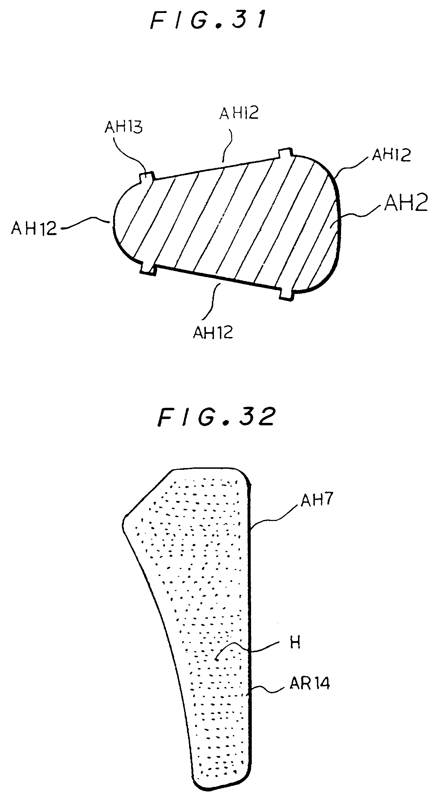

Fig. 31 is a sectional view taken on line III-III of Fig. 30. The cross section is symmetrical and comprises flat surfaces with a width of about 18 mm, curved surfaces with radius curvatures of 6 and 25 mm and widths of 10 and 14 mm, and the bank-shaped projections AH13 used to partition the surfaces.

Fig. 32 is a plan view of the thin sheet AH7. The size of the external shape thereof is slightly smaller than that of the femoral stem porous lamination component accommodation section AH12. The thin sheet AH7 has a thickness of 100 µm and is provided with non-pore fringe portion AH14 with a width of about 1 mm around the entire circumference of the thin sheet AH7. In the other sections of the thin sheet AH7, a plurality of through holes H with an effective diameter of about 300 µm are provided.

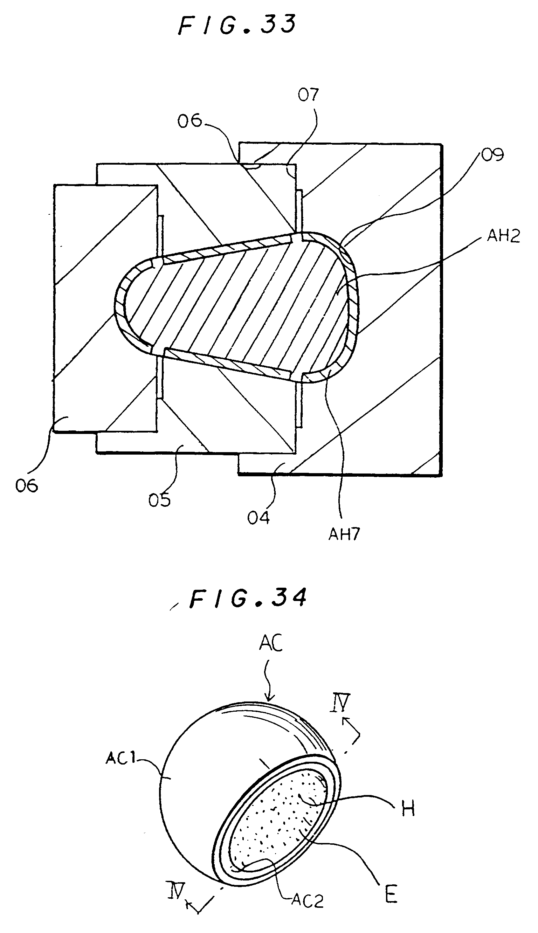

Fig. 33 shows a condition wherein a femoral stem porous lamination component AH16 is formed in the femoral stem AH2. Metal molds O4, O5 and O6 respectively correspond to the corresponding sections on the femoral stem porous lamination component accommodation section AH12. A form setting surface O9 is provided to form ten layers on the thin sheets AH7. Furthermore, stop surfaces O7 and O8 are provided so that the metal molds O4, O5 and O6 can be secured at the predetermined positions. When the metal molds are put in the vacuum furnace and heated at 900°C, the femoral stem porous lamination component AH16 can be formed in the femoral stem AH2. The porous lamination component AH16 can also be formed as described below. After a lamination component comprising thin sheets is heated to have a porous condition, it is cut by laser processing so that it can fit in the femoral stem porous lamination component accommodation section AH12, then it is formed by using the above-mentioned metal molds, thereby obtaining the porous lamination component AH16. When the porous lamination component is formed to have a curved surface, it can be bent beforehand in accordance with the curvature of the surface.

Fig. 34 is a perspective view on a femoral head cup AC on the present invention. The femoral head cup AC is a hemisphere with a diameter of 38 to 60 mm. It comprises a 2 mm thick sliding component AC1 made of PVA (polyvinyl alcohol) on the outside thereof and a 2 mm thick porous lamination component AC2 having a three-layer structure described below on the inside thereof. Accordingly, the cup has a thickness of 4 mm and functions as a sliding member used to slide with the acetabulum after the cup is replaced with the femoral head.

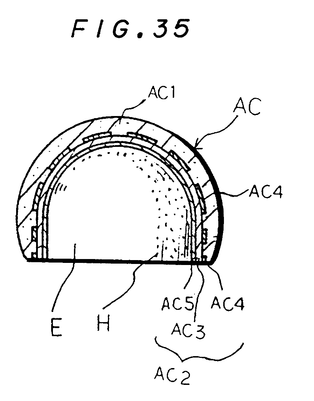

Fig. 35 is a sectional view taken on line IV-IV of Fig. 34. The above-mentioned porous lamination component AC2 has a three-layer structure comprising a PVA joint section AC4 having through holes H with an effective diameter of about 3 mm and being filled with PVA on the entire surface thereof and made by laminating five pieces of 100 µm thick pure titanium sheets, a bone contacting section AC5 having through holes H with an effective diameter of 300 µm on the entire surface thereon on the side of internal space E and made by laminating ten pieces of 50 µm thick pure titanium sheets, and a 1 mm thick cup component AC3 having no through holes, made on pure titanium and intervened between the PVA joint AC4 and the bone contacting section AC5.

Next, the method of making the above-mentioned femoral head cup AC is described below. First, thin sheets, each having through holes H with an effective diameter of 300 µm and a thickness of 50 µm, and other thin sheets, each having through holes H with an effective diameter of 3 mm and a thickness of 100 µm, were made by using the spherical surface unfolding method described for the example 4. Five pieces of the former thin sheets and five pieces of the latter thin sheets were laminated on both sides of the above-mentioned cup component AC3. These were temporarily secured and put into a metal mold (not shown). The metal mold was then put into the vacuum furnace and heated at about 900°C so that they were bonded. In this way, the above-mentioned porous lamination component AC2 was made.

Next, an appropriate amount of an adjusted PVA solution was poured into a metal mold (not shown). The above-mentioned porous lamination component AC2 was then placed on the mold, with the side of the internal space E facing upward. The metal mold and the porous lamination component AC2 were cooled for a while so as to fix PVA around the porous lamination component AC2, then put into silicone oil at 140°C for heat treatment to form the sliding component AC1 comprising PVA gel having a low water content on the outside of the porous lamination component AC2, thereby obtaining the above-mentioned femoral head cup AC.

The above-mentioned PVA solution was adjusted in accordance with the method disclosed by Japanese Laid-open Patent Application No. 2-86606 and No. 3141957 by adding 10 g of PVA having a polymerization degree of 5000 and a saponification degree of 99.9 mol % to a dimethylsulfoxide/water mixture solvent and by stirring the ingredients at 130°C nor two hours to dissolve them.

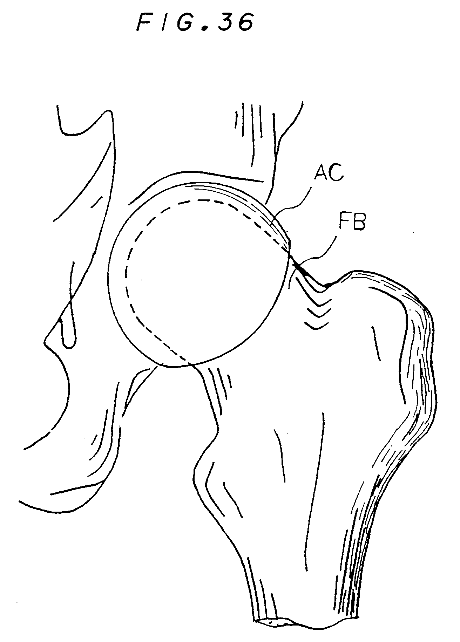

Fig. 36 shows a condition wherein the femoral head cup AC is fitted on the femoral bone FB.

As described above, the porous lamination components used to coat the surfaces of the prostheses can be integrated with not only the above-mentioned PVA but also organic materials such as superhigh molecular polyethylene or silicone to be used nor the sliding section members, impact load absorbing members or elastic deformation members of prostheses. The porous lamination components are, therefore, superior in enhancing the strength of the members made on organic materials and in joining the organic material members to bone tissues with which the organic material members make contact.

### (Example 5)

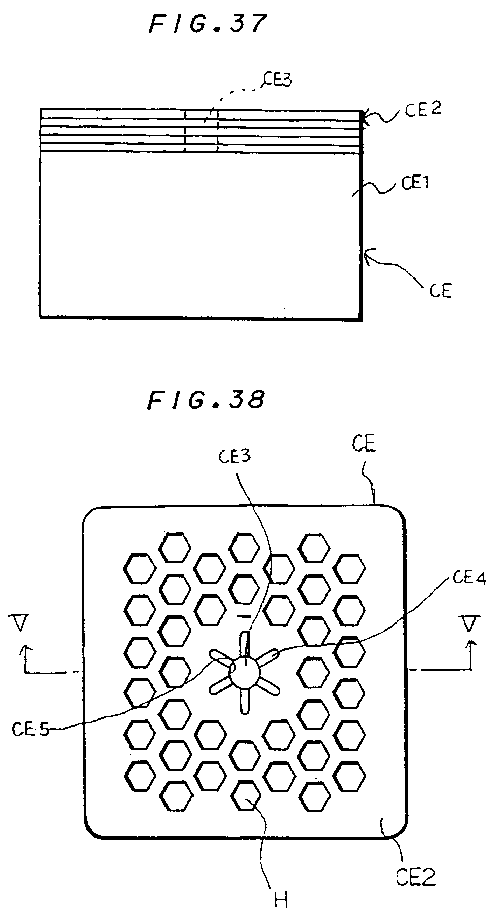

Fig. 37 is a side view of an artificial vertebral body CE used as a prosthesis of the present invention. The main body CE1 of this artificial vertebral body is a cubic component made of alumina ceramics, measuring 10 x 10 x 5 mm. On the end surface thereof, a porous lamination component CE2 having a thickness of 1 mm and made of titanium is integrated with the main body CE1. The porous lamination component CE2 comprises ten laminated thin sheets, each having a thickness on 100 µm.

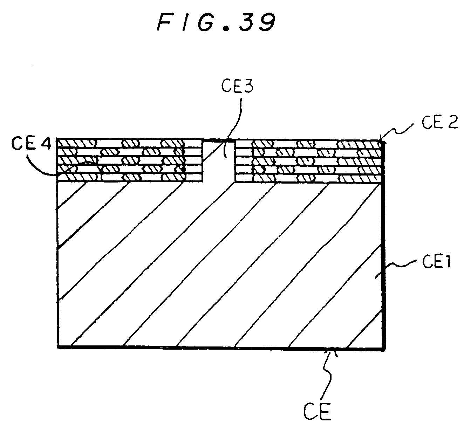

Fig. 38 is a top view of the artificial vertebral body CE used as a prosthesis of the present invention. The porous lamination component CE2 has most densely arranged through holes H with an effective diameter of 300 µm to form three-dimensional pores as shown in Fig. 39. At the almost central section of the end surface of the main body CE1, a joint-use cylinder CE3 measuring 0.98 mm in height and 2 mm in diameter is projecting. Around the internal circumference of the above-mentioned porous lamination component CE2, six deformation absorbing grooves CE4 are disposed so as to enclose the joint-use cylinder CE3. The pressure-fit surface CE5 making contact with the joint-use cylinder CE3 has a circular shape with a diameter of 2.3 mm. When each titanium thin sheet is placed on the end surface of the main body CE1, the pressure-fit surface CE5 functions to firmly make contact with the joint-use cylinder CE3.

Fig. 39 is a sectional view taken on line V-V of Fig. 38. The above-mentioned porous lamination component CE2 is supported and secured by the joint-use cylinder CE3 of the main body CE1. This assembly is then heated to about 900°C in the vacuum furnace so that the titanium thin sheets causes diffusion bonding. When paste including titanium particles is applied to the end surface of the above-mentioned main body CE1, the titanium particles combine with the metallic atoms of the porous lamination component CE2. In addition, the melted titanium on the thin sheets penetrates the small through holes disposed in the alumina ceramics of the main body CE1 at the end surface thereon to provide an anchoring effect. As a result, the joint between the porous lamination component CE2 and the main body CE1 is further enhanced.

The prosthesis of this example can be widely applied to the structures for surfaces making contact with bone tissues, such as femoral bone components of artificial knee joints made of ceramics, tibia components, iliac bone spacers, cranial bone prostheses and rib pins.

The prosthesis on the present invention can be firmly joined to living tissues by allowing the surrounding living tissues to promptly enclose the surfaces of the prosthesis after replacement and to penetrate the pores in the porous lamination component of the prosthesis and grow in the pores, thereby enhancing the durability of the prosthesis. Consequently, the present invention can provide prostheses which are not required to be replaced again, thereby imposing a less burden to patients.

## Claims

1. A prosthesis for the replacement of hard tissues of human bones and joints comprising a porous lamination component (S1) of metal thin sheets (1 - 20) each having a plurality of through holes (H) and a thickness of 150 microns or less, and being unharmful to the living body, said porous lamination component being formed such that said sheets are laid over one another and then diffusion-bonded therebetween into one body by heating so that said through holes communicate with one another in the direction of the thickness thereof, and the volume porosity of the prosthesis, namely the ratio of the volume of pores to the entire volume of the porous component thereof, is at least 45%.

2. A prosthesis according to claim 1 wherein the said through holes in one or more intervening layers are especially larger than those of the other layers (Fig. 10).

3. A prosthesis according to claim 1 wherein the diameters of the said through holes are such as to increase towards one surface of the lamination component or the other (Figs. 8, 9).

4. A prosthesis according to claim 1, 2 or 3 wherein the metal thin sheets have hexagonal through holes (H).

5. A prosthesis according to claim 1, 2 or 3 wherein the said wall of each through holes has a central raised portion (h).

6. A prosthesis for the replacement of hard tissue of human bones and joints according to any one of claims 1 to 5, wherein the prosthesis is entirely of said porous lamination component.

7. A prosthesis for the replacement of hard tissue of human bones and joints according to any one of claims 1 to 5, wherein the prosthesis is partially composed of said porous lamination component at a determined surface portion of the prosthesis base being made of metal, inorganic material to fabricate artificial bones, joints or other prosthesis.

8. A method of making a prosthesis for the replacement of hard tissues of human bones and joints comprising the following steps of:
perforating metal thin sheets each having a thickness of 150 µm or less to provide a plurality of through holes, and being unharmful to the living body;
laying said metal thin sheets thus obtained over one another so that said through holes may communicate with one another in the direction of the thickness thereof; and
diffusion-bonding between said metal thin sheets into one body by heating so as to form a porous lamination component for the prosthesis whereby a prosthesis having a volume porosity, namely the ratio of the volume of pores to the entire volume of the porous component thereof of at least 45% is obtainable.

9. A method according to claim 8 wherein the metal sheets are perforated by an etching method.

10. A method of making a prosthesis for the replacement of hard tissues of human bones and joints according to claim 8 or claim 9, wherein said laminating and diffusion-bonding take place at a determined surface portion of the base of the prosthesis of an artificial bone or joint.

## Patentansprüche

1. Prothese für das Ersetzen von harten Geweben menschlicher Knochen und Gelenke, umfassend eine poröse Schichtungskomponente (S1) aus Metalldünnplatten bzw. -folien (1-20), von denen jede eine Mehrzahl von Durchgangslöchern (H) und eine Dicke von 150 Mikron oder weniger hat, und unschädlich für den lebenden Körper ist, wobei die poröse Schichtungskomponente derart ausgebildet ist, daß die Platten bzw. Dünnplatten bzw. Folien übereinander angeordnet und dann untereinander durch Erwärmen bzw. Erhitzen zu einem Körper diffusionsgebunden sind, und zwar so, daß die Durchgangslöcher miteinander in der Richtung der Dicke derselben in Verbindung sind, und die Volumenporosität der Prothese, nämlich das Verhältnis des Volumens der Poren zu dem gesamten Volumen der porösen Komponente derselben, wenigstens 45% ist.

2. Prothese gemäß Anspruch 1, worin die Durchgangslöcher in einer oder mehreren dazwischenliegenden Schichten speziell größer als jene der anderen Schichten sind (Figur 10).

3. Prothese gemäß Anspruch 1, worin die Durchmesser der Durchgangslöcher derart sind, daß sie nach der einen Oberfläche der Schichtungskomponente oder der anderen zu zunehmen (Figuren 8, 9).

4. Prothese gemäß Anspruch 1, 2 oder 3, worin die Metalldünnplatten bzw. -folien hexagonale Durchgangslöcher (H) haben.

5. Prothese gemäß Anspruch 1, 2 oder 3, worin die Wand von jedem der Durchgangslöcher einen mittigen erhöhten Teil (h) hat.

6. Prothese für das Ersetzen von hartem Gewebe menschlicher Knochen und Gelenke gemäß irgendeinem der Ansprüche 1 bis 5, worin die Prothese vollständig aus der porösen Schichtungskomponente ist.

7. Prothese für das Ersetzen von hartem Gewebe menschlicher Knochen und Gelenke gemäß irgendeinem der Ansprüche 1 bis 5, worin die Prothese teilweise zusammengesetzt ist aus der porösen Schichtungskomponente in einem bestimmten Oberflächenteil der Prothesenbasis, die aus Metall, anorganischem Material hergestellt ist, um künstliche Knochen, Gelenke oder eine andere Prothese herzustellen.

8. Verfahren zum Herstellen einer Prothese für das Ersetzen von harten Geweben menschlicher Knochen und Gelenke, umfassend die folgenden Schritte:
Perforieren von Metalldünnplatten bzw. -folien, von denen jede eine Dicke von 150 µm oder weniger hat, um eine Mehrzahl von Durchgangslöchern vorzusehen, und die unschädlich für den lebenden Körper sind;
Anordnen der auf diese Weise erhaltenen Metalldünnplatten bzw. -folien übereinander so, daß die Durchgangslöcher in der Richtung der Dicke derselben miteinander in Verbindung sein können; und
Diffusionsbinden zwischen den Metalldünnplatten bzw. -folien zu einem Körper durch Erwärmen bzw. Erhitzen, so daß eine poröse Schichtungskomponente für die Prothese ausgebildet wird, wodurch eine Prothese, die eine Volumenporosität, nämlich das Verhältnis des Volumens der Poren zu dem gesamten Volumen der porösen Komponente derselben, von wenigstens 45% hat, erhältlich ist.

9. Verfahren gemäß Anspruch 8, worin die Metallplatten bzw. -folien durch ein Ätzverfahren perforiert werden.

10. Verfahren zum Herstellen einer Prothese für das Ersetzen von harten Geweben menschlicher Knochen und Gelenke gemäß Anspruch 8 oder Anspruch 9, worin das Schichten und Diffusionsbinden in einem vorbestimmten Oberflächenteil der Basis der Prothese eines künstlichen Knochens oder Gelenks stattfinden.

## Revendications

1. Prothèse pour le remplacement de tissus durs d'os humains et d'articulations humaines, comprenant un composant stratifié poreux (S1) formé de minces feuilles métalliques (1-20) possédant chacune une pluralité de trous traversants (H) et ayant une épaisseur de 150 micromètres ou moins et étant inoffensives pour le corps vivant, ledit composant stratifié poreux étant formé de telle sorte que lesdites feuilles sont déposées les unes sur les autres, puis réunies entre elles par diffusion avec chauffage pour former un corps de telle sorte que lesdits trous traversants communiquent dans le sens de leur épaisseur, et la porosité volumique de la prothèse, c'est-à-dire le rapport du volume des pores à l'ensemble du volume du composant poreux de la prothèse, est égale au moins à 45 %.

2. Prothèse selon la revendication 1, dans laquelle lesdits trous traversants situés dans une ou plusieurs couches intercalaires sont nettement plus grands que ceux des autres couches (figure 10).

3. Prothèse selon la revendication 1, dans laquelle les diamètres desdits trous traversants sont tels qu'ils augmentent en direction d'une surface ou de l'autre surface du composant stratifié (figures 8,9).

4. Prothèse selon l'une des revendications 1,2 ou 3, dans laquelle les feuilles métalliques minces possèdent des trous traversants hexagonaux (H).

5. Prothèse selon la revendication 1, 2 ou 3, dans laquelle ladite paroi de chacun des trous traversants comporte une partie centrale surélevée (h).

6. Prothèse pour le remplacement du tissu dur d'os humains et d'articulations humaines selon l'une quelconque des revendications 1 à 5, dans laquelle la prothèse est constituée entièrement par ledit composant stratifié poreux.

7. Prothèse pour le remplacement d'un tissu dur d'os humains et d'articulations humaines selon l'une quelconque des revendications 1 à 5, dans laquelle la prothèse est partiellement constituée dudit composant stratifié poreux dans une partie de surface déterminée de la base de la prothèse formée d'un métal, d'un matériau minéral pour fabriquer des os artificiels, des articulations artificielles ou d'autres prothèses.

8. Procédé pour fabriquer une prothèse pour le remplacement de tissus durs d'os humains et d'articulations humaines comprenant les étapes suivantes consistant à :
percer des feuilles métalliques minces possédant chacune une épaisseur de 150 µm ou moins pour former une pluralité de trous traversants et inoffensives pour le corps vivant;
disposer les unes sur les autres lesdites feuilles métalliques minces ainsi obtenues de telle sorte que lesdits trous traversants peuvent communiquer entre eux dans la direction de leur épaisseur; et
réunir par diffusion, avec chauffage, lesdites feuilles métalliques minces pour former un corps de manière à former un composant stratifié poreux pour la prothèse, ce qui permet d'obtenir une prothèse ayant une porosité volumique, c'est-à-dire le rapport du volume des pores à l'ensemble du volume du composant poreux de la prothèse, égale au moins à 45 %.

9. Procédé selon la revendication 8, selon lequel les feuilles métalliques sont perforées au moyen d'un procédé d'attaque chimique.

10. Procédé pour fabriquer une prothèse pour le remplacement de tissus durs d'os humains et d'articulations humaines selon la revendication 8 ou la revendication 9, selon lequel ladite stratification et ladite liaison par diffusion s'effectuent dans une partie de surface déterminée de la base de la prothèse d'un os artificiel ou d'une articulation artificielle.
